# EUROPEAN PATENT APPLICATION

(11) **EP 3 462 394 A1**
(43) Date of publication of application: **03.04.2019**
(21) Application number: 18207119.1
(22) Date of filing: 01.12.2014
(51) Int. Cl.: G06Q 10/10, G16H 10/60, G16H 50/20, G06F 16/28, G06F 16/22, G06F 16/11

(54) **SYSTEM FOR CONVERTING NATIVE PATIENT DATA FROM DISPARATE SYSTEMS INTO UNIFIED SEMANTIC PATIENT RECORD REPOSITORY SUPPORTING CLINICAL ANALYTICS**

(30) Priority: 29.11.2013 US 201361910253 P
(62) Divisional of application: 14865827.1
(71) Applicant: Plexina Inc., Calgary Alberta T2E 8W1 (CA)
(72) Inventor: BALUTA, Wasyl, Calgary, Alberta T2E 8W1 (CA)
(74) Representative: Williams, Ceili

(57) **Abstract**

Clinical Information Systems (CIS) may combine evidence-based and best-practice diagnosis and treatment of care delivery into an electronic workflow with clinical decision support (CDS) tools (e.g. order sets, structure documentation, rule-based alerts, compliance and performance reports) to improve care delivery. Configurating CIS's has required manually intensive processes and clinical experts to determine mapping of clinical concepts in the CDS to the CIS. Changes in medicine change configuration requirements causing changes to data structure, semantics, and even historical information. Traditional mapping is non-explicit and hard coded with a high effort cost for reconfiguration, interoperability, and quality assessment. Using Semantic Context Maps, simultaneously mapping naturally stated, native encoding, and semantic encoding in a clinical context, provides simplification of configuration of CDS and increases value of clinical information as medicine evolves. The invention constructs semantically encoded clinical data using semantic context mapping to enable analytics using semantic processing instead of traditional query analytics.

## Description

The present invention relates to a system and a process for converting at least a portion of an unencoded raw patient record into a validated coded record using Semantic Context Maps.

### Background of the Invention

### Key Parts of a Clinical Information System

When indexing the patient record some elements may come from an electronic Clinical Information System ("**CIS**") with discrete structure and may be relatively natively or proprietarily coded, even if associated with a standard. The CIS objects generally used in the care of patients include observations, interventions, problems, symptoms, and rules, but other types of objects might be used in specific CIS implementations. By way of further example:
An observation might include an assessment, diagnostic or lab result, a symptom, some aspect of the patient's history, and so on;
An intervention might typically represent an order for some action to be performed on the patient, e.g. feed them a specific diet, perform a lab or diagnostic test, administer a medication, walk for 5 minutes, and so on;
A rule might typically represent a simple or compound predicate of observations or interventions or other aspects of care and then some action, e.g. "IF blood pressure elevated AND has stroke symptoms THEN patient condition is urgent." The CIS can interpret when to evaluate the rule, what user interface warnings or communications are initiated when the patient condition state changes;
Clinical problems might include a disease, disorder, or set of symptoms if there is no specific cause identified.
A symptom may be anything that is a sign of something irregular in a patient's body: pain, fever, limited range of motion, changes in skin, irregular levels of various components of the body, discolouration, inflammation, change in cognitive behaviour or personality, and so on. Every system or anatomical part of the body can break down and the body's reaction to the breakdown can present in the patient as one or a series of symptoms. There are seemingly countless symptoms, as there are numerous disorders. Determining a root cause, (i.e. assessment and diagnosis) and attempting to return the patient to normal health (i.e. treatment) or highest quality of life is the essence of the modern practice of medicine.

Ultimately the CIS captures data about a patient for all these components or objects and forms a native encoded patient record, even if the internal terminologies are standards-based.

The native records from one CIS are rarely, if ever, directly portable to another CIS due to even minute differences in detailed native coding, CIS configuration, representation, and terminology, data or database structure, or ordering, labeling, or semantic intent of any or all of the record components of the CIS relatable to a patient ID.

### CIS Capabilities, Standardized Practice and Configuring a CIS

A CIS is essentially a configurable information system used in association with the delivery of care and management of patients in various environments. Electronic Medical Record Systems ("**EMRS**"), Electronic Health Record Systems ("**EHRS**"), and Practice Management Systems ("**PMS**") are all types of CIS for different care delivery environments. Lab Information Systems and Diagnostic/Imaging Systems typically manage tests and documentation of tests for patients, and, in a modern enterprise, will communicate back results to an over-arching EMRS or EHRS, and may be integrated to communicate and update bidirectionally with the EMRS or EHRS CIS. Pharmacy management systems and medication administration systems similarly interoperate or are integrated with EMRS and EHRS to provide a unified management experience for physicians, and a single source record at least within the care environment relevant to the CIS. Regardless of branding, most of the systems are modularized if integrated, and most systems have many modules to support the various areas of care. Sometimes the modules are all part of one system, or at least deployed together, and other times involves components from various CIS's sharing only key identifier information about the patient.

The CIS typically can be adapted or configured to accommodate and support care of patients with various conditions, under various environments, with varying clinical workflows. Generally, a CIS enables certain convenient capabilities during the care delivery process optimized for a particular environment with standardized clinical knowledge and some best practice guidance embedded in its design.

For example, to document a patient's condition the CIS may have structured documentation templates specialized for various conditions. The CIS will typically offer a lookup of historical information. Actively intelligent CIS systems may present documentation that is relevant to a particular situation based upon observations related to a current patient encounter, or historical information. However, most current CIS systems are simple passive systems that allow clinicians to lookup information they need from convenient search or navigation areas, but with reasonably well linked information to current diagnostic and test results related to a particular object of interest at the time, and to a particular patient ID.

More modern CIS systems may be heavily configured to provide actionable guidance using clinical order sets.

Order sets are diagnostic and treatment intervention templates created for convenience or for prescriptive treatment protocols. Order sets can be relatively simple involving for example, under 10 interventions, e.g. Convenience Analgesics, to rather complex systems involving 100 - 250 interventions, e.g. treatments in oncology, transplant surgery recovery, critical care, and so on.

Rules may also be configured in the system to automatically alert a clinician of important status changes in patient condition, important clinical workflow steps, or something that may predictably impact patient potential for positive outcome, or potentially dramatically complicate care, or impact on cost efficiency.

All of these components can be considered Clinical Decision Support ("CDS") tools. Each of these CDS tools of a modern CIS may change with discoveries in the science and practice of medicine. As new, better ways to care for patients are discovered, even new CDS tools will be introduced. As such, the configuration of the CIS may be adapted to meet the functional and informational needs of the CDS.

### Plexina Platform Background

Over the last decade the applicant has developed infrastructure for next generation Clinical Decision Support management, Clinical Knowledge Management, and more recently, DevOpS for healthcare-the integration of development, operations, and services for modern healthcare with integrated service delivery via an integrated CIS or set of CIS.

The clinical knowledge components ("CKC") representing parts of CDS tools in applicant's novel system effectively form a knowledge base of executable electronically actionable, evidence or best-practice based CDS tools-in essence, clinical source code.

### Summary of the Invention:

In applicant's system, known as the Plexina Platform, the process of converting readily available clinical knowledge sources into deployable executable components for a CIS, is largely automated.

The core elements of that system include a transformation tool that maps natural language clinical concepts into a standardized terminology and structure, and Semantic Context Maps (SCMs) that simultaneously capture a standardized semantic description (terminology and structure) with one or more native CIS translations. That is, the SCMs represent a semantically consistent representation of the various clinical knowledge and CDS components that are deployed in associated CISs.

The current invention is a new component for leveraging the Plexina Platform during CIS implementation to create an analytic capability that is more powerful than traditional data analytics. Specifically, the system of this invention can infer a comprehensive unified semantic patient record from non-semantically encoded patient record data both from a single source CIS, or from a diverse system of CISs managing patients across a continuum of care.

The current invention leverages SCMs for implementation of each clinical knowledge component (CDS tool) and bi-directional interaction with an associated CIS.

In an embodiment, clinical designers may create abstract semantic designs of the clinical knowledge components, called clinical knowledge models ("CKM") of the CIS with a design tool, and deploy those CKM to one or more native systems. That is, the CKM and the underlying SCM has sufficient translation details to be directly deployed into various CIS. The SCM needs to contain simply references to CIS element types and key identifier information, though additional deployment or representation information may be stored in the SCM.

By analogy, this development can be likened in the field of software development to a software Integrated Development Environment which allows the developer to develop in one language, and deploy runtime executable objects to one or more different application server platforms, for example Apache with JBOSS, and Microsoft Application Server.

The present invention provides a computer-based system and a computer-implemented process operating on an application server platform for automated analysis of patient records by first converting at least a portion of a semantically unencoded native CIS patient record into a validated semantically coded record.

The system of the present invention is configured to facilitate and automate the encoding of patient information in a data set that is not encoded to a particular terminology and structure.

The system aims to provide an accelerated means to accurately encode unencoded clinical data relative to a clinical context for evaluation against a clinical indicator scenario such as a care plan or clinical study. Today, without the process or system of this invention, such studies involve manual case reviews over the course of months by a clinical researcher.

In one aspect, the process of the present invention comprises the steps of (i) semantically encoding care assessments and care plans; (ii) semantically indexing data relatable to a patient ID; (iii) creating a Semantic Analytic Profile that represents a query construct and includes at least one Query Semantic Context Record within the Semantic Analytic Profile to provide an evaluation summary of the Semantically Encoded Patient Record; and (iv) comparing Semantically Encoded Patient Records to the at least one Query Semantic Context Record within the Semantic Analytic Profile to provide an evaluation summary of the Semantically Encoded Patient record. Step (i) of the process is implemented by using a Semantic Coding Viewer / Editor installed on the application server platform to create Semantic Context Maps. Step (ii) of the process semantically indexes data from the natively-encoded patient record from the CIS using the Semantic Context Maps to produce a Semantically Encoded Patient Record in which every observation and invention of the Semantically Encoded Patient Record is associated with one or more Semantic Context Records. This step uses a collocation index of semantic concepts, synonyms, ancestors, descendants and links to relevant Semantic Context Maps.

Each SCM comprises at least one identifier of a clinical knowledge component representing part of a Clinical Decision Support tool, such as care plan assessment questions, interventions, and clinical indicator scenarios or criteria of the CIS. Each SCM further comprises at least one Semantic Context Record, including a correlated set of (a) an identifier, using one or more clinical terms, of at least one part of a Clinical Decision Support tool; (b) at least one standardized semantic concept; and c) at least one identifier of an element of the CIS. The correlated set establishes a relationship between the element of the CIS and the Semantic Context Record and a context for the element is established by the Semantic Context Map.

In one embodiment, the system encodes the patient information semantically by using the semantic context maps (SCMs) used during deployment. This enables automating 100% validated semantic encoding. Alternative mechanisms using NLP would still require manual validation. The SCMs maintain semantic concepts from a standard clinical ontology or nomenclature (SNOMED CT or UMLS for example), or other terminologies.

In another embodiment, the system encodes the patient information semantically by using the SCMs used during deployment but augments this with alternative mechanisms using NLP where no SCM exists, e.g. when free form text fields were used for data entry thereby otherwise requiring manual validation. The NLP however is enriched to use the contexts in the SCMs and commonality analysis to determine applicability of the SCM to the particular patient record.

In a further embodiment, the Semantic Analytic Profile also includes at least one time period; and at least one rule, with at least one predicate for indication of a semantic concept. In such an embodiment, the Semantic Analytic Profile is used with a Semantic Evaluator. The Semantic Evaluator comprises a rule engine that uses semantic symbols and evaluates symbol matching within an ontology. The Semantic Analytic Profile further includes directives to control semantic flexibility across relationships using synonyms, ancestry or descendants.

In another embodiment, the process has the added step of comparing the Semantic Context Records of Semantically Encoded Patient Records with a Query Semantic Context Record within a Semantic Analytic Profile to provide another result which includes an evaluation summary of the semantically encoded patient record. In yet another embodiment, the process has an added step of comparing Semantically Encoded Patent Records to other Semantically Encoded Patient Records to create clusters of Semantically Encoded Patient Records associated by commonality as a precurser to the step added in the preceding sentence.

In yet another embodiment, there is added the step of displaying the result of the match strength and explanation of a comparison of Semantically Encoded Patient Records to a Semantic Analytic Profile.

In another aspect, the invention provides a computer-based subsystem that comprises: (i) a Semantic Coding Viewer / Editor installed on the application server platform; (ii) a semantic indexer; (iii) a semantic contextualizer; (iv) a semantic comparator/evaluator; and (v) a repository. The Semantic Coding Viewer / Editor semantically encodes care assessments and care plans to create Semantic Context Maps. The semantic indexer is arranged to semantically index some natively-encoded data relatable to a patient ID accessed from the clinical information system using a collocation index of semantic concepts, synonyms, ancestors, descendants and links to relevant Semantic Context Maps. The semantic contextualizer is arranged to use Semantic Context Maps to identify the semantic equivalent to a natively coded object or groups of objects which form care assessments and care plans of the clinical information system. The semantic contextualizer is further arranged to generate a Semantically Encoded Patient Record in which every observation and intervention of the Semantically Encoded Patient Record is associated with one or more Semantic Context Record. The semantic comparator/evaluator receives a Semantic Analytic Profile that represents a query construct and includes at least one Query Semantic Context Record. The semantic comparator/evaluator compares some of the semantically indexed data relatable to a patient to the at least one Query Semantic Context Record within the Semantic Analytic Profile. The repository stores the Semantically Encoded Patient Records, selected depending on the outcome of the comparison of the semantic comparator / evaluator: either being identified as candidate patient cases for analytics or to evaluate compliance of care with evidence and/or best practice care.

In an embodiment, the invention comprises a subsystem for use in relation to one or more CISs for converting at least a portion of an unencoded patient record which includes data relatable to a patient ID maintained in a CIS, the data comprising objects or elements natively coded to suit a CIS, the objects or elements being observations, interventions, problems, symptoms, rules or other data, into a validated semantically coded record, the sub-system itself having: a semantic indexer for semantically indexing some data relatable to a patient ID accessed from a CIS; a semantic contextualizer for semantically indexing objects or groups of objects or elements which form core assessments or care plans to generate a semantic context record; a semantic commonality comparator for comparing records to records to create clusters of common records relevant to the semantic context records; a semantic comparator/evaluator for comparing at least some of the semantically indexed data relatable to a patient ID to at least some of the semantic context records which may be within a semantic analytic profile; and a compliance validator for generating an indication of match strength and an explanation of comparison of semantically indexed data relatable to a patient ID to the core assessments or care plans in the Query Semantic Context Record, which may be within a Semantic Analytic Profile.

In one embodiment, the semantic comparator/evaluator is for comparing at least one Semantically Encoded Patient Record from the repository to an analytic profile. The semantic comparator/evaluator includes at least one Semantic Analytic Profile containing native concepts mapped to semantic concepts, evaluation expressions, and directives. The semantic comparator/evaluator finds an initial match based on commonality set comparison of concepts in the Semantic Analytic Profile to concepts in the Semantically Encoded Patient Record for all candidate Semantically Encoded Patient Records, a semantic rule evaluator evaluating all rules in the Semantic Analytic Profile and inferring the conclusions.

In another embodiment, the semantic comparator/evaluator compares a Semantic Context Map to a Best-Practice Semantic Analytic Profile. The Best-Practice Semantic Analytic Profile is based on best-practice standards compliant with evidence-based quality measures. The semantic comparator/evaluator further includes (i) at least one Best-Practice Analytic Profile; (ii) one or more Semantic Context Maps from a library of Semantic Context Maps; (iii) an initial match; and (iv) a semantic rule evaluator. The Best-Practice Analytic Profile is a type of clinical knowledge model, containing native concepts mapped to semantic concepts forming the Query Semantic Context Record, evaluation expressions, and directives. The initial match is based on commonality set comparison of concepts in the Best Practice Semantic Profile to concepts in the Semantic Context Maps.

In a further embodiment, any corrected validated Semantically Encoded Patient Record is compared using the semantic comparator, and produces details used by the semantic contextualizer and semantic indexers to refine matching and inference algorithms.

In a further embodiment, the invention comprises the conversion of at least a portion of an unencoded patient record which includes data relatable to a patient ID maintained in a CIS, the data comprising objects or elements natively coded to suit a CIS, the objects or elements being observations, interventions, problems, symptoms, rules or other data relevant to a patient or treatment or the CIS environment, into a validated semantically coded record; the process steps include at least: semantically indexing some data relatable to a patient ID accessed from a CIS; semantically indexing objects or elements which form core assessments or care plans within the CIS or patient care environment to generate a semantic context record; comparing records to records to create clusters of common records relevant to the semantic context records; comparing at least some of the semantically indexed data relatable to a patient ID to at least some of the semantic context records, which may be within a semantic analytic profile, and generating an indication of match strength and an explanation of comparison(s) of semantically indexed data relatable to patient ID(s) to the core assessments or care plans in the semantic context record.

### Description of the Figures/Drawings

Figures 1a, 1b and 1c are block-diagrams showing an overview of the operations in components of the system of the invention
Figure 2 is a block diagram describing functions and steps in functional Module 1 of the invention (building a semantically encoded clinical data index)
Figure 3 is a screen shot depiction of a data structure Context Map for a CKC of the system
Figure 4 is a block diagram describing using the semantic comparator to identify semantic patient records
Figure 5 is a block diagram describing using the semantic evaluator

### Detailed Description of the Invention

### MODULE 1: Semantic CDR Constructor System

The current invention is a software application that can construct a Semantic Clinical Data Repository ("SCDR") that is semantically accurate without the augmentation of CIS and in an automated way in situations such as where the Plexina Platform was used to deploy a CKM into a CIS.

Using the SCMs of the CKM in the reverse, the system can identify the CKM used in the delivery of care and form a Semantically encoded Patient Record (SPR).

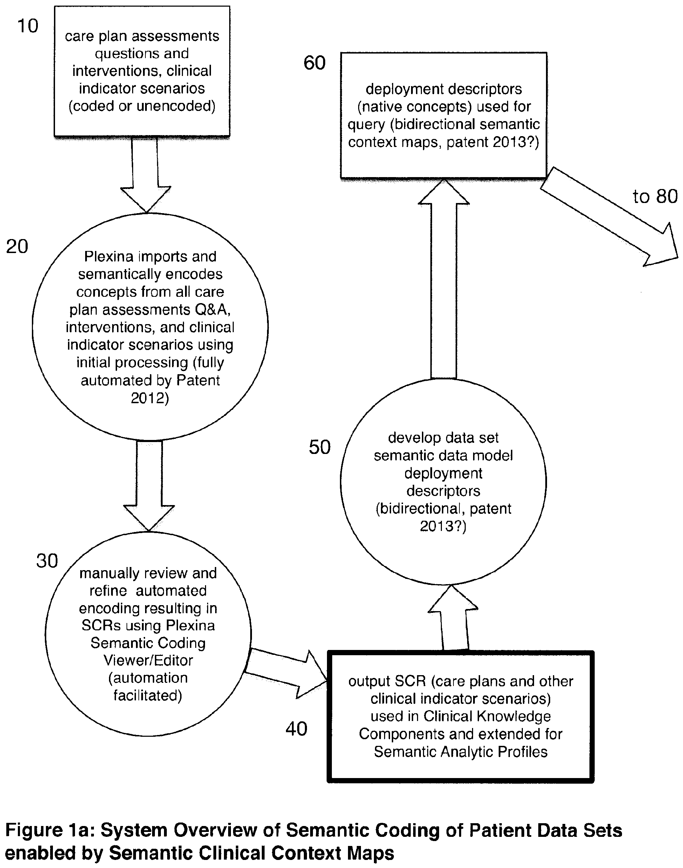

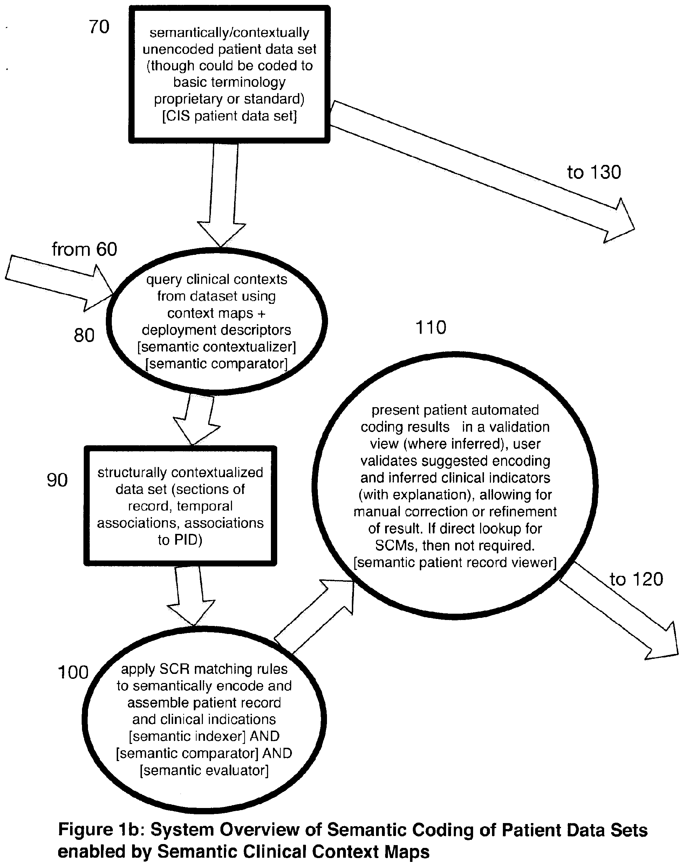

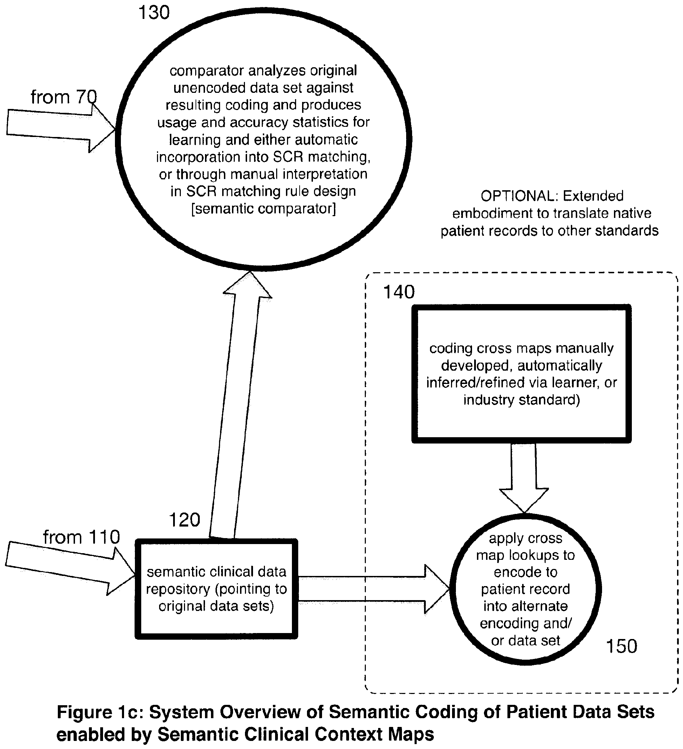

### Figures 1a, 1b and 1c - An overview of one embodiment of this invention

Clinical Knowledge Components 10 such as care plan assessment questions, interventions, and clinical indicator scenarios or criteria are usually represented in free form text, or natural text description.

A prior implement of the Plexina Platform 20 automatically encodes the clinical knowledge components into native CIS terminologies and/or to semantic terminologies, creating a framed Semantic Context Map ("SCM") with a semantic context record ("SCR") to represent a single concept. If the natural description has a direct mapping to the semantic ontology then that is considered a simple semantic concept ("SSC"). If the natural description requires several semantic concepts or a structure to represent it, then it is considered a complex semantic concept ("CSC"). A simple semantic context map can be created for all natural concepts and/or native concepts that are independent or unassociated with a broader clinical context. For clarity, a SCM can contain one or more SCRs.

The SCM's automated encoding is validated and refined 30 using a Semantic Coding Viewer/Editor if necessary to create a semantic context record.

The Semantic Coding Viewer/Editor is also used to add directives and evaluation rules to the SCM resulting in a Semantic Analytic Profile 40, a special type of SCM.

The SCMs 50 contain direct references to deployment concepts and then deployment descriptors for any particular CIS. Using the concept type details, a simple lookup is used to find the deployment descriptor 60 which determines how to configure the CIS with the particular concept in the SCM.

The Semantic Indexer 80 uses the Semantic Context Maps and deployment descriptors when encountering a native concept in the CIS native patient record data from the CSI native clinical data repository 70 to identify Semantic Context Record and semantic concept equivalents. The semantic context map may contain additional information around the context of care, such as stage of care, implied severity of condition, and so forth. Such information may also be semantically encoded as part of a semantic context record, but not natively deployed, thereby enriching the semantic capabilities of the CIS.

The result of the semantic indexing and contextualization is a semantically contextualized patient record 90, whether anonymous, or identifiable, with or without links back to the original native patient record details.

The Semantic Comparator and the Semantic Evaluator 100 are used in conjunction to semantically analyze the semantically encoded patient data according to semantic analytic profiles ("**SAP**") defined by the semantic evaluation rules. The Semantic Context Records ("SCR") are a part of the SAP representing the simple or complex semantic concepts. SAPs are used to represent the query constructs for advanced purposes such as determining compliance with care standards, compliance with evidence, consideration for quality reporting, or for very specialized research.

The Semantic Comparator identifies that concepts of one source record (e.g. SAP), are relevant or related to the other source record (e.g. a semantic patient record). The Semantic Comparator may handle direct and indirect relationships against a variety of dimensions, depending on the purpose of the analysis. For example, related concepts include synonyms, ancestry or descendants along the IS-A relationship, or other relationships in the ontology to enrich the experience further, such as disorders and "symptoms of".

The Semantic Evaluator incorporates a rule engine that may use semantic symbols and evaluate symbol matching with the flexibility of the ontological hierarchy. The SAP may contain directives to control the semantic flexibility across relationships such as "EXACT" or "DESCENDENT OF" or "ANCESTOR OF", e.g. evaluate rules for drug classes instead of enumerating exact drugs, routes and dose forms. As with traditional rule engines, this invention allows for new conclusions to be inferred.

The Semantic Patient Record Viewer 110 enables the semantically encoded patient record to be viewed, along with inferred concepts, and may optionally include links back to the native patient record for review and/or validation. This review does not need to be used if the patient record semantics were directly looked up from the SCM rather than inferred with NLP.

The finalized semantic patient records are collected into a repository 120, which can be a database, file based, or index based repository.

In an extended embodiment, the Semantic Comparator 130 may compare semantics identified automatically for the patient record, with the semantics refined in the semantic patient records viewer, to leverage those statistics to refine the ranking algorithm it uses during searching, indexing or evaluation.

In another embodiment, cross maps 140 from the semantic ontology to other standards can be used to translate SPR to other standard terminologies and yield a translated patient record 150. This may be applied to translate natively encoded records into a HL7 CCDA, for example, or another standard.

Also, if multiple CIS systems are deployed to, SCMs from one system can be re-mapped to another SCM where the SCRs match, or by creating new SCMs for the CKM.

The invention extends a prior implementation to represent clinical indicators and clinical criteria.

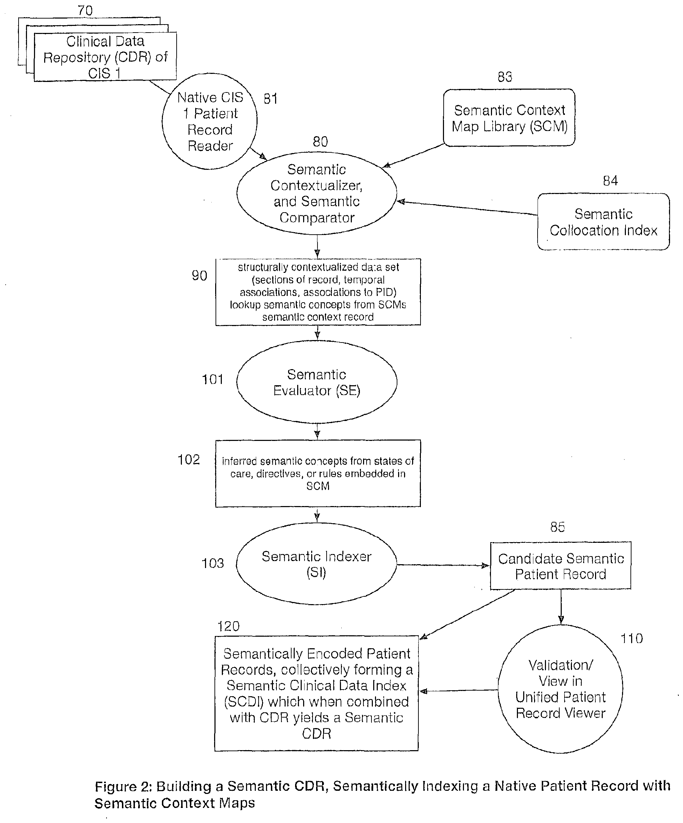

### Figure 2 - Building A Semantic Clinical Data Repository, Semantically Indexing a Native Patient Record with Semantic Context Maps

Original patient record data 70 coded to native system, CIS. Observations, intervention and other documented elements or objects of a patient record can exist in one or more CIS.

Semantic Context Map of a CKM 83. Encoded semantically and natively for patient observation sets (structured documentation) and interventions (order sets and orders) and/or combinations of other patient care flow elements and CDS tools.

A semantic collocation index 84 contains simple semantic concepts, linked with synonym concepts, ancestors, and descendants. Additionally contains various attributes for categorizations to support various processing algorithms. Also contains links to relevant SCMs for contextualization. This function may be provided by any of a number of different types of separate subsystems.

Semantic Contextualizer 80 uses the SCM to reverse lookup the semantic representation of observations documented about a patient encounter, and the interventions performed on the patient from one or more SCMs. In the event the patient record was documented from standardized documentation templates and intervention templates, the native concept has sufficient detail to identify from which CKM (and thus from which related SCM) the observations or interventions originate. Specifically an observation assessment form or order set might be tied by name or id to a particular CKM that was used in the native system.

In the event the observations or interventions were manually selected from outside the standard templates, the Semantic Contextualizer may utilize a commonality comparison (a semantic comparator) of all native concepts against the library of CKMs. The commonality comparison algorithm identifies the SCMs that have the most items in common with some extensions for optional vs. mandatory data, and resolving a series of documented/timestamped events into an independent or series of encounters.

Free form text fields for objects such as observations or interventions are analyzed with natural language processing in an embodiment using synonyms, ancestors, descendants, and even SCMs. Inferred semantic concepts from free form text are flagged for clinical validation (may be done manually with an automation facilitated user interface) in a Patient Record Viewer.

The native observation or intervention will contain links to its source CDS object and thus making it easy for the Semantic Contextualizer to identify the correct SCM to use. Semantic concepts along with identifying patient information (whether anonymous or linked to actual patient data) are copied into the initial semantic patient record 90.

The SCMs 101 that apply for the patient record may contain evaluation rules for the Semantic Evaluator to evaluate and infer new concepts and enrich the semantic patient record further 102.

The Semantic Indexer 103 collects and organizes all patient record segments for the same patient, and indexes all the semantic details, with links to native CIS or CDR, and SCMs. The repository can be database based, file system based, index, or other type data persistence technology.

The semantic indexer (SI) allows the indexing of data relatable to a patient ID in an unencoded or natively or proprietarily coded patient record.

The semantic indexer may semantically index one or more of the following details from a patient record:
- episode and/or event
- sections
- observations and interventions
- fields of observations or interventions
- values within a field
- potentially delimited by periods, punctuation, or by lines.

The semantic indexer indexes native concepts to the most granular atomic simple semantic concept (SSC) record that directly represents each native concept, for example, in a simple case a single SNOMED CT concept can represent an order or observation. In the SCM, however, a native concept may be represented by one or more simple semantic concepts (SSC), creating a complex semantic concept (CSC) record. CSCs can be treated like expressions, lists, sets, or other common collections can be treated in software programming, allowing for various levels of sophistication of electronic processing instructions or actions.

Any semantic concepts inferred for a semantic patient record 85 from the process may need review and validation. The Semantic Patient Record Viewer 110 can present the information in a usable way, highlighting inferred concepts with explanations of how the values were concluded. The user can correct or discard inferences. If the semantic patient record did not include any inferred values, but rather looked up values, then the semantic patient record viewer and review is not required.

The repository of semantically encoded patient records is the semantic CDR 120. The semantic processing state can be captured with each record if necessary. The Semantic CDR 120 is effectively the semantic processing results on the patient record with key information related to the source native patient record CDR or CIS. The semantic CDR can be updated periodically before analysis, or updated when semantics change in the SCM.

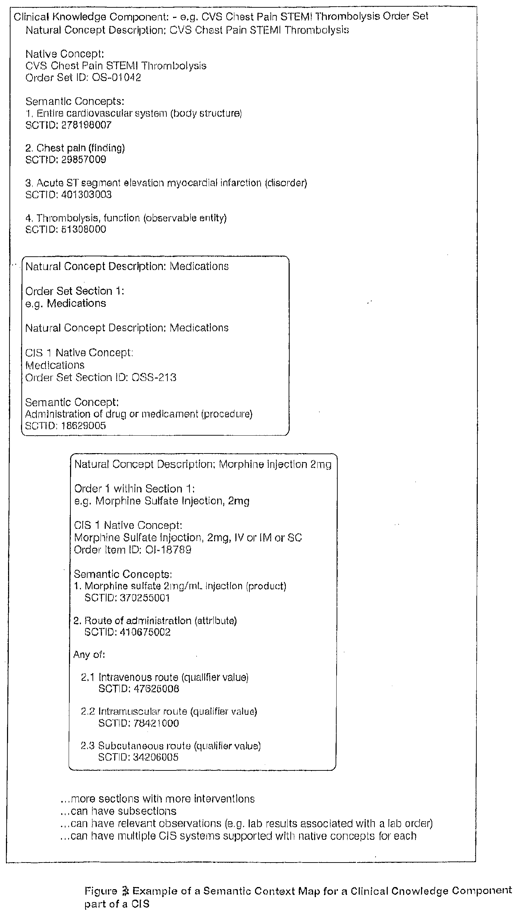

### Figure 3 - A Semantic Context Map (SCM) representing part of a Clinical Knowledge Model, i.e. a segment of an Order Set for STEMI Treatment

The SCM allows for a consistent semantic representation and multiple concurrent native representations simultaneously.

For example, if attempting to find related CSCs, a set or list comparison of the individual SSCs within the CSC, either exact or partial match, can be identified with a common pattern discovery algorithm.

As a second example, by treating the CSC record as an expression using individual semantic concepts (NLP can identify AND, OR, NOT, etc.) the CSC may then represent a rule or question. Rules may also be involved in comparison of semantic concepts to find related or relevant rules to other SSCs or CSCs. Rules can also be leveraged in an analytic system, or inference engine.

The semantic contextualizer NLP algorithms can use information from several dimensions for processing, and ultimately lookup:
- all equivalent SSCs, i.e. concepts from the ontology and their synonyms
- all ancestors of the SSC along an "IS-A" type relationship
- all descendants of the SSC along an "IS-A" type relationship
- other details or arbitrary categorizations that may be used for specific processing algorithms such as determination of stage of care, temporal relevance, or scope limiting values for limiting flexible searches

In another embodiment, a semantic contextualizer may also include:
- any related order set templates and the orders, and the equivalent SSCs or CSCs (for orders, sections, and other components of the order set template) contained within, i.e. the SCMs within the CKM for the order set type clinical knowledge components
- any related structured documentation templates and the observations, and the equivalent SSCs or CSCs (for objects such as observations, sections, and other components of the structured documentation template) contained within the SCMs within the CKM for the structured documentation type clinical knowledge components
- any complex semantic concepts CSC derived from the SCMs and then directly or indirectly related SSCs or CSCs.

The relationship of a clinical concept, simple or complex, to the SCMs adds a level of context to each concept that is unique to this invention.

Specifically, observations and orders are now related to a context of care through the SCM. This directly enables the context to be maintained with the continuously maintained CKM which may be developed, without having to manually explicitly maintain the contexts hard coded in analytic queries, reports, or even a collocation index. That is, the semantic contextualization works automatically by processing the ontology and the library of CKM and linking the information in a way to achieve efficient lookups along every key concept dimension, including synonyms, semantic relationships and hierarchy, and clinical applications in the clinical workflow.

The contextualization data typically used for lookup can be pre-processed once from the key sources of information and organized into an efficient access structure over multiple servers. This index of key lookup information only needs to be updated when ontologies change, or when the SCM library changes.

### Using Commonality to Identify Implied Context

In another embodiment of the reverse lookup, when the context is unknown, or the patient experience is documented outside of standardized structured documentation templates and order set templates, a set of native concepts from the native patient record is compared to the set of native concepts in the library of the SCMs in the CKMs.

Every native concept has at a minimum one semantic concept record in the library of SCM, even if it is simple to represent. This ensures that any ad hoc observation that may be documented or intervention that may be ordered has SCM. This also allows the entire patient record to be completely semantically encoded.

The commonality algorithm used by the Semantic Comparator identifies the SCMs with the most overlap with the patient record's native concepts of interests. The commonality ranking or analysis can be simple in nature, based for instance, on frequency of instances of native concepts, or elaborate, for instance using historical record analysis to determine lists of likely disorders, and applicable CKMs for given symptoms, result ranges, or even completed treatments.

### MODULE 2: Semantic Analytic Comparison of Semantically Encoded Patient Records to a Semantically Encoded Clinical Analytic Profiles

This component of the system allows a clinical analyst user to design a special type of Clinical Knowledge Model for an analytic query called a Semantic Analytic Profile ("SAP") 302 and then evaluate that query against a semantically encoded patient record. Much like the CKM, a SAP contains a semantic structured set of concepts to discretely and unambiguously describe natural free form description of the analytic query.

This module of the invention, the semantic comparator, 300 at figure 3, can be used for identifying candidate patient cases for analytics. Using another instance of the Semantic Evaluator 400, the system can evaluate compliance of care with evidence and/or best-practice care, or infer patient state from the semantics of discrete native data captured in the patient record.

The semantic concepts in the SAP are also SSC or CSC records and can also be in the collocation index for accelerated processing. The SAP can represent concepts such as (by example):
e.g.1. A brain scan was performed within 120 minutes of onset of stroke symptoms
e.g.2. The patient was given smoking cessation therapy prior during the encounter
e.g.3. The patient was administered ace inhibitors

As in Figure 2, the SAP can be represented as a collection of semantic concepts, either as a set, list, or expression form for electronic processing. Furthermore, the SAP can involve stages of care, indicators such as observations documented or interventions requested and actually delivered, a temporal order in which concepts or objects occurred or were ordered to occur, and so on.

Using the first example, semantically in the CIS there may be (for example) 20 brain scans that could possibly be performed. However, the number of brain scans suitable for stroke diagnosis might be 4. Traditional analytic engines would have to encode all 4 options specifically into a traditional SQL query, combined with SQL criteria to determine if the patient was experiencing stroke symptoms, in the fashion or structure that the relevant CIS presented those (checkbox, dropdown option, free form text) and so forth. Without the invention, there were two options for this type of compliance analysis:
1) a manual case review effort by clinical researchers finding patient cases using indicators (if available) to identify candidates for research and then validating the criteria
2) criteria with all possible combinations hard coded into queries for the CIS which typically takes months to create a single meaningful report.

The evaluation criteria are subject to change along with the evolution of medical science and so manual research needs to be re-performed, and hard coded reports have to be re-developed.

Using a second example of smoking cessation, the patient record could contain indicators of anything from a prescription, to education on breaking the smoking addiction, to specific lab tests.

As medical science evolves, semantic representation and interpretation can also evolve and change for native concepts. The invention enables a re-evaluation of the data by simply refining the SAP and re-running the evaluation. In traditional analytics applications, based on SQL or other mainstream database, and even nouveau object databases or no SQL, the semantic representation is effectively hard coded in the native representation of the query and the data. With changes to medicine, the analytics queries have to be re-developed. With data warehouses, the data has to have views and dimensions re-built, and even schema potentially re-normalized. This is impractical for enterprise health CIS and data infrastructures.

With the invention of this application, changes to semantic interpretation of what is currently best-practice are simply updated in a CKM and SAP, and the semantic indexer re-indexes those concepts that were changed. Further, the SAP might not need changing and now just finds new records that meet the criteria, or evaluates to the new results appropriate to the new semantics.

Essentially with the CKM any clinical observation or intervention involved in a care workflow-be it a simple set of a few orders, or a very complex care pathway across a continuum of care like hip and knee replacement surgery or even chronic care situations-can be represented, deployed and documented. Now, with the semantic SAP and semantic analytic comparison, complex clinical analytic queries can be performed in real-time, to identify patient cases that meet criteria, and also evaluate patient cases according to criteria.

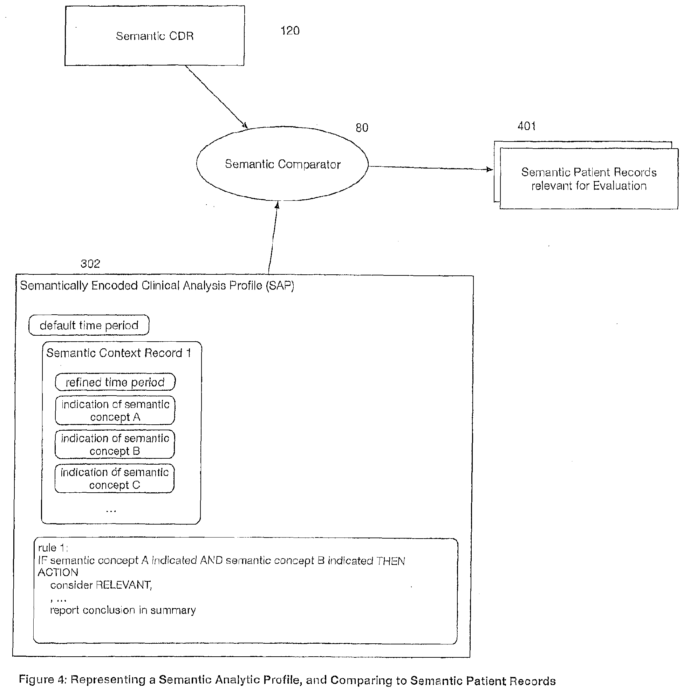

### Figure 4: Representing a Semantic Analytic Profile, and Comparing to Semantic Patient Records

The Semantic Analytic Profile 302 defines the semantic concept indicators, and clinical stages of care or time scope for consideration. Also any rules with directives to consider relevant or irrelevant would be included.

The Comparator 400 uses a commonality comparison combined with processing of directives or rules to determine the candidate set of patient records that should be included in further analysis 401.

The Semantic Patient Records 401 is a set of records relevant for Evaluation, as identified by The Comparator 400.

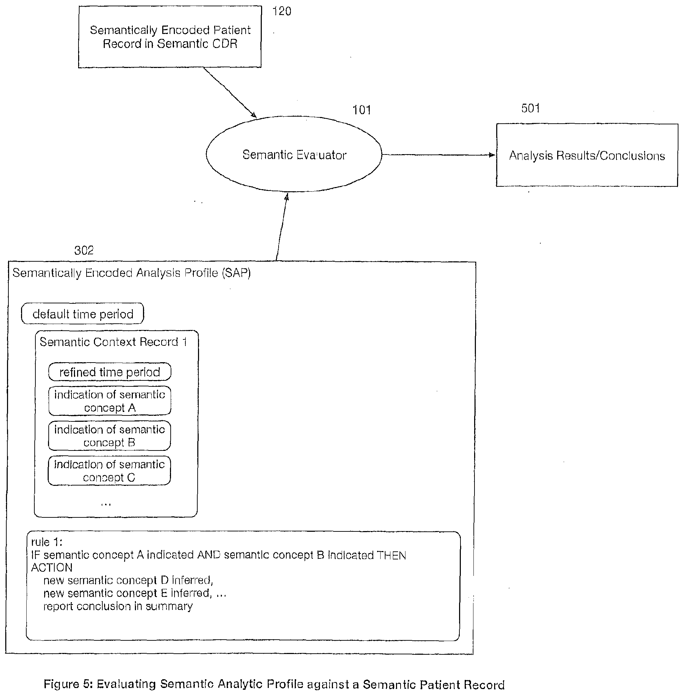

### Figure 5: Evaluating Semantic Analytic Profile against a Semantic Patient Record

The repository 120 of all Semantic Patient Records is stored in the Semantic CDR.

The Semantic Analytic Profile 302 contains an Analytic Query Model which includes semantic concepts, directives and operators. Directives can be instructions (such as) to conclude whether proper care or improper care is given an expression involving semantic concepts "indicated[COMPLIANT] if semantic concept A FOLLOWS concept B present WITHIN 120 minutes of ADMISSION" or "indicated [NON-COMPLIANT] if semantic concept C present AND semantic concept D not present by NEURO SPECIALIST BEFORE DISCHARGE". The concepts A, B, C, or D can be simple semantic concepts or complex semantic concepts. "indicated[COMPLIANT]" is a directive to infer this record is COMPLIANT with the compliance criterion. Other directives can be used according to purpose of analytics.

The Semantic Evaluator 500 loops through all semantically encoded patient records (one or many, which are in scope for analysis) first looking for simple set comparison for presence of equivalent semantic concepts. Second level evaluation determines the type of directives, such as COMPLIANT, or NON-COMPLIANT or other conclusion that may be inferred simply by the presence of a semantic concept and evaluation of the logical expressions of the rules. The last step is to produce a report, create a result set in a database, or publish results for some other similar result production or consumption process.

The Semantic Patient Evaluation Summary 501 is a record for semantic patient record evaluated of which criteria were in the analytic evaluation profile, and the inferred results from the evaluation of the criteria.

Subsequent processes can use the evaluation results from the semantic evaluation to produce reports, invoke alerts to the care team, trigger other real-time or offline processes, or be collected for population health studies (for example).

### Benefits of the Invention

Automated semantic indexing has great benefits for clinical analytics such as population health and clinical effectiveness research. Traditionally, population health and clinical effectiveness researchers design criteria for candidacy and evaluation.

The candidacy criteria define the scope of patients and patient cases that should be considered for research. The research criteria are criteria that need to be evaluated on the candidate cases. Traditionally, this is a largely manual effort where a clinical researcher works with database administrators to attempt to write many ad hoc queries to determine the candidates, and then more ad hoc queries to provide the research results. Such a research project would typically take 6-12 months, and cost $100,000's.

Furthermore, the research is fragile semantically. The researcher must identify all combinations of various observations and elements that may apply, and construct queries that expand the combinations. For example, the query of "suspected stroke patient received a head scan" needs to be analyzed in great detail to understand the context of data being captured in the CIS, and clinician workflow associated with documenting the information in the CIS. The data might be clear and easily documented where a suspected disorder is captured, e.g. stroke, and the intervention is named "head scan for stroke". But very likely that will not be the normal case. Stroke might have been suspected, and the physician may not have documented that fact in the CIS, but may have sent the patient for a "CT of head" anyway, but could have also ordered "MRI of brain", "CT of brain", "MRA", and so on. The researchers enumerate the combinations of ways information could have been captured, manually review all the patient records, evaluate the criteria, and tally up the results. Then they may realize, for their particular research question, that they should have included a "CTA" scan. Then the researchers must re-evaluate the cases, or may even have to go back and find additional candidate cases. And so on.

This re-analysis may also need to be performed if new research appears that changes the research hypotheses or method.

With the invention, selection of candidate cases for research and evaluation of criteria are simple semantic queries. The expansion of semantics to similar or relevantly related information is automated and eliminates the need to design complex queries that must change when medical science and the research objective and/or data that needs to be analyzed changes.

Furthermore, with clinical data warehouses, query-able dimensions are designed by normalizing disparate data sources, and designing views for important perspectives. In this process many assumptions and tradeoffs are made on semantics and structure with the clinical data elements. However, in medicine, the science of best practices changes, and thus the reporting needs and data warehouse structure may change frequently. Keeping a denormalized repository yields more stability when changes occur, but requires more work to get specific information for reporting. A highly normalized system is practically nearly impossible to maintain.

Using this invention with the semantic-to-native mapping, as semantic context is altered by system or CIS configurators or designers, the historical information can be semantically indexed again, effectively, yielding a new semantic interpretation of the same native data. This can be critical for research and downstream clinical analytics as the understanding of medical science evolves, and retrospective re-evaluation of existing data in an automated fashion.

In addition, reports that need to consider one more observation or intervention would simply be a modification to the SAP. The semantic comparator and semantic evaluator would pick up on the new inclusion because of compatible semantics, and thus include the new case in the research results.

The previous description of the disclosed embodiments is provided to enable any person skilled in the art to make or use the present invention. Various modifications to those embodiments will be readily apparent to those skilled in the art, and the generic principles defined herein may be applied to other embodiments without departing from the spirit or scope of the invention. Thus, the present invention is not intended to be limited to the embodiments shown herein, but is to be accorded the full scope consistent with the claims, wherein reference to an element in the singular, such as by use of the article "a" or "an" is not intended to mean "one and only one" unless specifically so stated, but rather "one or more". All structural and functional equivalents to the elements of the various embodiments described throughout the disclosure that are known or later come to be known to those of ordinary skill in the art are intended to be encompassed by the elements of the claims. Moreover, nothing disclosed herein is intended to be dedicated to the public regardless of whether such disclosure is explicitly recited in the claims.

## Claims

1. A computer-implemented process operating on an application server platform for automated analysis of patient records by first converting at least a portion of a natively encoded patient record from a Clinical Information System (CIS) into a validated semantically encoded patient record, the process comprising the steps of:
(i) Semantically encoding care assessments and care plans, using a Semantic Coding Viewer / Editor installed on the application server platform, to create Semantic Context Maps, each Semantic Context Map comprising:
(a) at least one identifier of a clinical knowledge component, each clinical knowledge component representing at least a part of a Clinical Decision Support tool such as care plan assessment questions, interventions and clinical indicator scenarios or criteria of the CIS; and
(b) at least one Semantic Context Record, wherein the Semantic Context Record includes a correlated set of the following:
A) an identifier, using one or more clinical terms, of at least one part of a Clinical Decision Support tool;
B) at least one or more "standardized semantic concepts"; and
C) at least one identifier of an element of the CIS;
whereby the correlated set establishes a relationship between the element of the CIS and the Semantic Context Record and a context for the element is established by the Semantic Context Map; and then
(ii) Semantically indexing data relatable to a patient ID from the natively-encoded patient record from the CIS using the Semantic Context Maps to produce a Semantically Encoded Patient Record in which every observation and intervention of the Semantically Encoded Patient Record is associated with one or more Semantic Context Records, this step using a collocation index of semantic concepts, synonyms, ancestors, descendants and links to relevant Semantic Context Maps; and then
(iii) creating a Semantic Analytic Profile that represents a query construct and includes at least one Query Semantic Context Record; and then
(iv) comparing Semantically Encoded Patient Records to the at least one Query Semantic Context Record within the Semantic Analytic Profile to provide an evaluation summary of the Semantically Encoded Patient record.

2. The process of claim 1, wherein the Semantic Analytic Profile includes, in addition to the at least one Query Semantic Context Record:
(a) at least one time period; and
(b) at least one rule with at least one predicate for indication of a semantic concept; and
the Semantic Analytic Profile is used with a Semantic Evaluator, which comprises a rule engine that uses semantic symbols and evaluates symbol matching within an ontology, the Semantic Analytic Profile including directives to control semantic flexibility across relationships using synonyms, ancestry or descendants.

3. The process of claim 1, with the added step of comparing Semantically Encoded Patient Records to other Semantically Encoded Patient Records to create clusters of Semantically Encoded Patient Records associated by commonality.

4. The process of claim 1, with the added step of comparing the Semantic Context Records of Semantically Encoded Patient Records with a Query Semantic Context Record of a Semantic Analytic Profile.

5. The process of claim 1, adding a step after step (iv) of displaying the result as the match strength and explanation of a comparison of Semantically Encoded Patient Records to Semantic Analytic Profile.

6. The process of claim 5, adding a step of receiving user input with respect to the match strength and explanation, and storing the user input.

7. A computer-based sub-system operating on an application server platform that is arranged to convert at least a portion of a natively-encoded patient record which patient record includes data relatable to a patient ID maintained in a Clinical Information System (CIS), the data comprising objects natively coded to suit the Clinical Information System - the objects being observations, interventions, problems, symptoms, care assessments, care plans, rules or other data - into a validated semantically coded record, the sub-system comprising the following:
(i) A Semantic Coding Viewer / Editor installed on the application server platform, to semantically encode care assessments and care plans, to create Semantic Context Maps, each Semantic Context Map comprising:
(a) at least one identifier of a clinical knowledge component, each clinical knowledge component representing at least a part of a Clinical Decision Support tool such as care plan assessment questions, interventions and clinical indicator scenarios or criteria of the CIS; and
(b) at least one Semantic Context Record, wherein the Semantic Context Record includes a correlated set of the following:
(A) an identifier, using one or more clinical terms, of at least one part of a Clinical Decision Support tool;
(B) at least one or more "standardized semantic concepts"; and
(C) at least one identifier of an element of the CIS;
whereby the correlated set establishes a relationship between the element of the CIS and the Semantic Context Record and a context for the element is established by the Semantic Context Map;
(ii) A semantic indexer component arranged to semantically index some natively-encoded data relatable to a patient ID accessed from the clinical information system using a collocation index of semantic concepts, synonyms, ancestors, descendants and links to relevant Semantic Context Maps;
(iii) A semantic contextualizer component arranged to use Semantic Context Maps to identify the semantic equivalent to a natively coded object or groups of objects which form care assessments and care plans of the clinical information system and to generate a Semantically Encoded Patient Record in which every observation and intervention of the Semantically Encoded Patient Record is associated with one or more Semantic Context Records;
(iv) A semantic comparator/evaluator component arranged to receive a Semantic Analytic Profile that represents a query construct and that includes at least one Query Semantic Context Record and to compare some of the semantically indexed data relatable to a patient to the at least one Query Semantic Context Record within the Semantic Analytic Profile;
(v) A repository arranged to store the Semantically Encoded Patient Records , those selected depending on the outcome of the comparison of the semantic comparator / evaluator: either being identified as candidate patient cases for analytics or to evaluate compliance of care with evidence and / or best practice care.

8. The sub-system of claim 7 with a compliance validator for generating an indication of match strength and an explanation of a comparison of semantically indexed data relatable to a patient to the core assessments and care plans in the Query Semantic Context Record within the Semantic Analytic Profile.

9. The subsystem of claim 7 where Semantic Context Maps can be first determined for input to the semantic indexer by a performing a commonality comparison using native concepts in the comparison in another semantic commonality comparator.

10. The sub-system of claim 7 with an added semantic rule evaluator where a result summary is produced for the result of an evaluation expression, said evaluation expression comprising rules involving semantic concepts as field and value symbols in the precedent:
(i) Where the rule evaluator evaluates a precedent with logical and mathematical expressions, unit conversions, and functions and if the expression is true may perform a list of actions from a list of antecedent actions; and may infer a plurality of conclusions which may instantiate a new semantic value symbol; and
(ii) Where the resolution of semantic symbols is done using lookup from an ontology for related concepts, either synonyms for equivalence, or ancestors and descendants along ontological relationships in a collocation index.

11. The sub-system of claim 7 wherein the semantic comparator/evaluator component is for comparing at least one Semantically Encoded Patient Record from the repository to an analytic profile and includes:
(i) At least one Semantic Analytic Profile containing native concepts mapped to semantic concepts, evaluation expressions, and directives; and
(ii) is arranged to find an initial match based on commonality set comparison of concepts in the Semantic Analytic Profile to concepts in the Semantically Encoded Patient Record for all candidate Semantically Encoded Patient Records, a semantic rule evaluator being arranged to evaluate all rules in the Semantic Analytic Profile and infer the conclusions.

12. The sub-system of claim 7 wherein the semantic comparator/evaluator component is for comparing a Semantic Context Map to a Best-Practice Semantic Analytic Profile, the Best-Practice Semantic Analytic Profile being best-practice standards compliant with evidence-based quality measures, and includes:
(i) At least one Best-Practice Semantic Analytic Profile being a type of clinical knowledge model, containing native concepts mapped to semantic concepts forming the Query Semantic Context Record, evaluation expressions, and directives;
(ii) One or more Semantic Context Maps from a library of Semantic Context Maps;
(iii) An initial match being based on commonality set comparison of concepts in the Best-Practice Semantic Analytic Profile to concepts in the Semantic Context Maps;
(iv) A semantic rule evaluator being arranged to evaluate all rules in the Best-Practice Semantic Analytic Profile and infer the conclusions.

13. The sub-system of claim 7, in which at least one Semantically Encoded Patient Record is reviewed, validated, and corrected if necessary using a semantic patient record viewer.

14. The sub-systems of claim 13, in which any corrected validated Semantically Encoded Patient Record is compared using the semantic comparator, and produces details used by the semantic contextualizer and semantic indexers to refine matching and inference algorithms.

15. The sub-systems of claim 13 in which, using cross maps from the semantic terminology to other standard or native system terminologies, a semantic patient record translator translates the Semantically Encoded Patient Record in the terminology of alternative standards supported by the cross map.
